(19) Europäisches Patentamt

European Patent Office

Office européen des brevets

(11) **EP 2 289 541 A1**

(12) **EUROPEAN PATENT APPLICATION**

(43) Date of publication:
**02.03.2011 Bulletin 2011/09**

(51) Int Cl.:
*A61K 38/49* (2006.01)      *C07K 14/705* (2006.01)
*A61P 25/00* (2006.01)

(21) Application number: **09011145.1**

(22) Date of filing: **31.08.2009**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(71) Applicant: PAION Deutschland GmbH
**52062 Aachen (DE)**

(72) Inventors:
• **Petersen, Karl-Uwe**
  **52072 Aachen (DE)**
• **Vivien, Denis**
  **14073 CAEN (FR)**

• **Ali, Carine**
  **14073 CAEN (FR)**
• **Lopez Atalaya, José**
  **14073 CAEN (FR)**
• **Roussel, Benoit**
  **14073 CAEN (FR)**
• **Hommet, Yannick**
  **14073 CAEN (FR)**

(74) Representative: **Von Renesse, Dorothea et al**
  **König Szynka Tilmann von Renesse**
  **Patentanwälte Partnerschaft**
  **Lohengrinstrasse 11**
  **40549 Düsseldorf (DE)**

(54) **Treatment of neurological or neurodegenerative disorders**

(57)     Use of a protein or peptide for the manufacture of a medicament for the treatment of neurological or neurodegenerative disorders, in particular stroke, wherein the protein or peptide is selected form the group consisting of a kringle protein or peptide comprising or consisting of amino acid sequences which are derived from the plasminogen activator-related kringle (PARK) domain or desmoteplase activator-related (DARK) domain.

**EP 2 289 541 A1**

**Description**

This invention relates to a method for the treatment of neurological or neurodegenerative disorders. More particularly, this invention relates to the protection of neurons and/or the blood brain barrier which are adversely affected as result of a neurological or neurodegenerative disorder.

Stroke is a leading cause of adult death and disability with approximately 6 million deaths per year, with an alarming projected incidence over the next decade due to the increase in the elderly population. Despite the significant advances that have been made in understanding the cellular and molecular pathophysiology of cerebral ischaemia, recombinant tissue-type plasminogen activator (rt-PA) remains the only approved acute treatment for ischaemic stroke. Hundreds of compounds have been tested so far in clinical trials for ischaemic stroke, but aside from rt-PA, none has proven effective.

The use of rt-PA is limited by a short therapeutic window (4.5 hours post-onset) and by promotion of both intracerebral haemorrhage and neurotoxicity. Thus, there is a critical need for safer and more effective treatments, in order to improve the global benefit of rt-PA induced thrombolysis and to provide treatment for stroke patients who are not eligible for thrombolysis (*i.e.* more than 80% of stroke patients).

T-PA is a serine protease with two faces which displays key roles in the intravascular space, at the interface between blood and brain and in the brain parenchyma (for review: Yepes et al., 2009). In the intravascular compartment, t-PA's main substrate is the inactive zymogen plasminogen and its main role is to promote fibrinolysis. Blood-derived t-PA can cross both the intact and the injured blood-brain barrier (BBB) (Benchenane et al. 2005a, Benchenane et al., 2005b) and thus can, together with endogenously produced t-PA, interact in the brain parenchyma with a variety of substrates, thus extending its functions above t-PA/plasmin(ogen)-driven extracellular matrix degradation.

There is a growing body of evidence indicating that the interaction between t-PA and the N-methyl-D-aspartate receptor (NMDAR), the low density lipoprotein receptor-related protein (LRP), annexin-II in glial cells and/or neurons activate cell signaling processes results in a deleterious outcome including cerebral edema, hemorrhagic transformation and cell death. Based on these multiple pathophysiological effects of t-PA, the participation of endogenous t-PA (supported by the side effects of exogenously applied rt-PA) is discussed beyond the established role in ischaemic disorders for several neurological or neurodegenerative disorders such as epilepsy, Alzheimer's disease, multiple sclerosis or meningitis.

According to the complex behaviour of t-PA several molecular strategies for inhibiting the deleterious effects of t-PA can be envisaged. However a strategy that can be transferred into the clinical situation is still missing.

Thus, it is the objective of the present invention to provide novel means for the treatment of neurological or neurodegenerative disorders, in particular of stroke.

This objective is solved by a method according to claim 1. Further embodiments of the invention are subject matter of additional independent or dependent claims.

The inventors have found that proteins comprising or consisting of a kringle domain are active at two t-PA relevant target sites: the blood brain barrier and the NMDA receptor and as a result exhibit vasoprotective and neuroprotective activity.

Accordingly, the kringle domain-containing proteins or peptides (hereinafter "kringle proteins" and/or "kringle peptide") are together referred to as "t-PA inhibitor".

As will be shown below the results from the animal model reveal that the t-PA inhibitors of the invention are not only effective when given together with t-PA but also when administered alone.

Thus, the invention relates to the use of a protein or peptide for the manufacture of a medicament for the treatment of neurological or neurodegenerative disorders, in particular stroke, wherein the protein or peptide is selected form the group consisting of:

(a) a kringle protein or peptide comprising or consisting of

(i) an amino acid sequence according SEQ ID NO:1, SEQ ID NO:2 or SEQ ID NO:3; or

(ii) an amino acid sequence with at least 70% identity, preferably 80%, more preferably 90% and most preferably 95% identity to the amino acid sequence given in SEQ ID NO:3; or

(iii) an amino acid sequence with at least 90% identity, preferable at least 95% or 100% identity to the plasminogen activator-related kringle (PARK) motif defined by the sequence: "CY-$X_3$-G-$X_2$-YRGTXS-$X_2$-ES-$X_3$-C-$X_2$-WNS-$X_2$-L-$X_4$-Y-$X_4$-PXA-$X_2$-LGLGXHNYCRNP-$X_4$-KPWCXVXK-$X_6$-EXC-$X_2$-PXC", wherein X denotes an arbitrary amino acid; or

(iv) an amino acid sequence with at least 92% identity, preferable at least 95% or 100% identity to the DARK motif defined by the sequence: "CY-$X_3$-G-$X_2$-YRGTXS-$X_2$-ESR-$X_2$-C-$X_2$-WNS-$X_2$-LXR-$X_2$-Y-$X_3$-MPXAFN-LGLGXHNYCRNPNXAXKPWCXVXK-$X_3$-F-$X_2$-ESC-$X_2$-PXC", wherein X denotes an arbitrary amino acid;

wherein said protein or peptide does not exhibit a serine protease activity.

The inventors could identify the kringle domain as the relevant domain for the inhibition of the t-PA transport through the BBB with the final proof that a protein consisting only of a plasminogen activator-related kringle (PARK) domain is also a t-PA transport inhibitor. Hence a protein or peptide comprising or consisting of this domain can be used as a vasoprotectant and/or neuroprotectant. This is not limited to a kringle domain of desmoteplase (Desmodus rotundus plasminogen activator) DSPA but applies also to the kringle domains of other plasminogen activators since it was found that kringle domains of different plasminogen activators were able to inhibit trans-BBB transport of rt-PA.

Hence the invention relates to the use of proteins with a plasminogen activator-related kringle (PARK) domain or a desmoteplase activator-related kringle (DARK) domain for the treatment of neurological disorders. In particular these proteins can be applied as vasoprotectants and/or neuroprotectants.

The protective activity of the kringle proteins is not caused by its protease activity. Hence the proteins of the invention do not exhibit the serine protease activity commonly shared by plasminogen activators.

This could be shown by the inventors in an *in vitro* model of BBB penetration. Herein, it was demonstrated that the transport of rt-PA can be blocked not only by the plasminogen activator desmoteplase but also by an inactivated so called 'clogged' DSPA (cDSPA). This opened the way to a new therapy approach since an inactivated DSPA will neither augment the plasminogen activating capacity of t-PA nor interfere with the plasminogen-activating and therefore clot-lysing efficacy of t-PA. Rather, it inhibit only the detrimental side effects of t-PA related thrombolytics. Furthermore, the inactivated DSPA is not capable of cleaving the NR1 subunit of the NMDA receptor and hence poses no risk of neurotoxicity on its own.

Furthermore the inventors could show that the co-incubation of rt-PA and clogged DSPA (or the isolated and purified kringle domain) had no toxic effect on the blood brain barrier.

A kringle is a triple looped polypeptide structure formed by three disulfide bonds. Kringles vary in length from about 79 to 82 amino acid groups. A high degree of sequence homology is shared among the single kringle of human urokinase (Günzler et al., 1982, Hoppe-Seyler's Z. Physiol. Chem. 363: 1155f), the two kringles of human tissue plasminogen activator (Pennica, et al. 1983, Nature 301: 214f), the two kringles of human prothrombin (Walz et al., 1977, PNAS 74: 1969f), and the five kringles of human plasminogen (Sottrup-Jensen et al. 1978, in "Progress in Chemical Fibrinolysis and Thrombolysis" (eds. Davidson et al.), 3: 191f). The relative positions of the six cysteines involved in the intra-kringle disulfide bridges are conserved in all kringles.

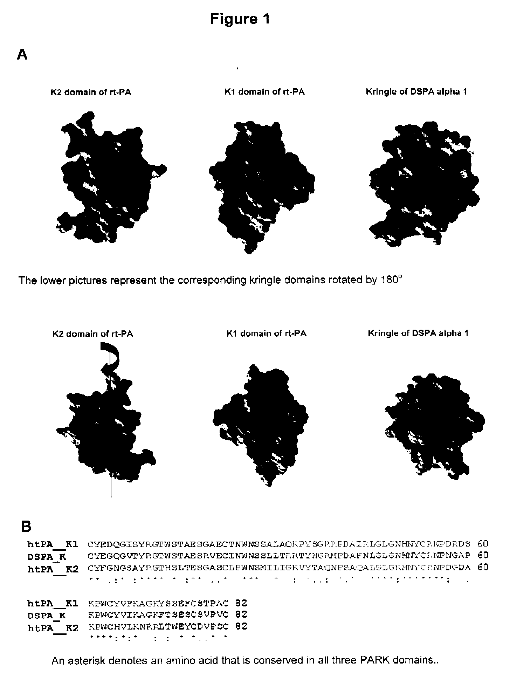

The term, "plasminogen activator related kringle domain" (PARK domain) as used in this application refers to proteins or peptides with an amino acid sequence with at least 90% identity, preferable at least 95% or 100% identity to the plasminogen activator-related kringle (PARK) motif defined by the sequence: "CY-$X_3$-G-$X_2$-YRGTXS-$X_2$-ES-$X_3$-C-$X_2$-WNS-$X_2$-L-$X_4$-Y-$X_4$-PXA-$X_2$-LGLGXHNYCRNP-$X_4$-KPWCXVXK-$X_6$-EXC-$X_2$-PXC", wherein X denotes an arbitrary amino acid (Fig. 1B).

This term in particular encompasses proteins comprising or consisting of the amino acid sequences SEQ ID NO:1, SEQ ID NO:2 and SEQ ID NO:3. It is also understood that polymorphic forms in the kringle region of these proteins may exist in nature where one or more amino acids may be added, deleted or substituted. Similar changes in a protein may also be brought about *in vitro* with the advent of the present day technology of point mutation of the gene or by chemical synthesis of the gene with any desired sequence. These modified structure(s) are therefore also included in the term, kringle(s), used in this application.

Sequences SEQ ID NO:1 and SEQ ID NO:2 show the amino acid sequences of the kringle 1 and 2 domains of the recombinant t-PA (alteplase). SEQ ID NO:3 shows the amino acid sequence of the kringle domain of desmoteplase (DSPA alpha 1).

>Seq ID No. 1: t-PA_kringle 1

CYEDQGISYRGTWSTAESGAECTNWNSSALAQKPYSGRRPDAIRLGLGNHNYCRNP
DRDSKPWCYVFKAGKYSSEFCSTPAC

>Seq ID No. 2 t-PA_kringle 2
CYFGNGSAYRGTHSLTESGASCLPWNSMILIGKVYTAQNPSAQALGLGKHNYCRNPD
GDAKPWCHVLKNRRLTWEYCDVPSC


>Seq ID No.3: DSPA_kringle
CYEGQGVTYRGTWSTAESRVECINWNSSLLTRRTYNGRMPDAFNLGLGNHNYCRN
PNGAPKPWCYVIKAGKFTSESCSVPVC


Other preferred kringle domains for said peptide or protein include a kringle domain from urokinase and prothrombin.

As used herein the" DARK domain" is defined as a peptide with an amino acid sequence of at least 90 % identity and preferable at least 95% or 100% identity to the following amino acid sequence motive resulting from the kringle sequence of DSPA alpha 1 as given in Figure 1B:


$$CY\text{-}X_3\text{-}G\text{-}X_2\text{–}YRGTXS\text{-}X_2\text{-}ESR\text{-}X_2\text{-}C\text{-}X_2\text{-}WNS\text{-}X_2\text{-}LXR\text{-}X_2\text{-}Y\text{-}X_3\text{-}MPXAFN\text{-}$$
$$LGLGXHNYCRNPNXAXKPWCXVXK\text{-}X_3\text{-}F\text{-}X_2\text{.}ESC\text{-}X_2\text{-}PXC;$$


wherein X denotes an arbitrary amino acid

In a further aspect of the invention the kringle protein consists of an amino acid sequence of either the DARK or the PARK domain as of Fig. 1B or Fig. 1A, respectively:


$$CY\text{-}X_3\text{-}G\text{-}X_2\text{–}YRGTXS\text{-}X_2\text{-}ES\text{-}X_3\text{-}C\text{-}X_2\text{-}WNS\text{-}X_2\text{-}L\text{-}X_4\text{-}Y\text{-}X_4\text{-}PXA\text{-}X_2\text{-}LGLGXHNYCRNP\text{-}}$$
$$X_4\text{-}KPWCHXVXK\text{-}X_6\text{-}EXC\text{-}X_2\text{-}PXC$$


or


$$CY\text{-}X_3\text{-}G\text{-}X_2\text{–}YRGTXS\text{-}X_2\text{-}ESR\text{-}X_2\text{-}C\text{-}X_2\text{-}WNS\text{-}X_2\text{-}LXR\text{-}X_2\text{-}Y\text{-}X_3\text{-}MPXAFN\text{-}$$
$$LGLGXHNYCRNPNXAXKPWCXVXK\text{-}X_3\text{-}F\text{-}X_2\text{.}ESC\text{-}X_2\text{-}PXC.$$


The kringle proteins of the invention preferably have a length of not more than 200 amino acids (aa), preferably not more than 150 aa, most preferably not more than 100 aa, and comprise the PARK or DARK motif as above. This has a length of 82 aa.

The t-PA inhibitor furthermore can include a lysine binding site, which is preferably defined by the presence of the amino acids Y36, W62 and H64, according to the numbering of the 82 aa kringle domain as used herein.

The t-PA inhibitor of the invention preferably inhibit the t-PA transport across the blood brain barrier (BBB) in mammals by at least 20%, preferably by at least 30%, most preferred by at least 40% or 50% assessable by the method described in Example A, Chapter 2 of the present invention.

Said t-PA inhibitor can bind specifically to the low-density lipoprotein (LDL) receptor-related protein (LRP). The LRP is a multifunctional endocytosis receptor that binds a variety of ligands, including t-PA, the β-amyloid precursor protein, alpha 2-macroglobulin, apolipoprotein E-enriched beta-very-low-density lipoprotein, and Pseudomonas exotoxin A, some of which are implicated in neurological diseases such as Alzheimer's disease. Due to LRP binding said protein or peptide can block the binding of t-PA or other ligands to the LRP thereby inhibiting the LRP-mediated transport over the BBB.

Furthermore, the t-PA inhibitor can bind to the NR1 subunit of the NMDA receptor. Due to this NR1 binding said protein or peptide can block the binding of other substrates or ligands to the NR1 subunit thereby inhibiting its proteolytical cleavage.

Due to the fact that said t-PA inhibitor does not bind to t-PA, therapeutically administered t-PA or other plasminogen activators will retain their normal thrombolytic function that is mandatory for a beneficial activity in thrombotic disorders.

Importantly, the inventors have shown that the kringle domain containing protein prevents t-PA-induced damage of the BBB (see Example 1).

Hence in one aspect of the invention the t-PA inhibitor can be used for the treatment of neurological or neurodegenerative disorders that are associated with enhanced permeability of the blood brain barrier. These disorders comprise ischaemic or thrombotic disorders such as stroke or TIA, epilepsy such as temporal lobe epilepsy (TLE), amyotrophic lateral sclerosis, multiple sclerosis, brain tumours, Parkinson disease, Alzheimer's disease, brain oedema, or CNS complications resulting from parasitic, bacterial, fungal or viral infections such as meningitis or encephalitis.

Based on these findings the invention relates to a method for the treatment of neurological and neurodegenerative disorders, particularly stroke, whereby the patient is treated with a therapeutically effective amount of a thrombolytic drug and an effective amount of a t-PA inhibitor. The invention further relates to a composite comprising a thrombolytic drug and a t-PA inhibitor.

Accordingly, said t-PA inhibitor can be used as an adjunct to a thrombolytic therapy, in particular for late t-PA-induced thrombolysis.

In a further aspect of the invention the t-PA inhibitor can be used for the acute or post-acute treatment of a neurological or neurodegenerative disorder, in particularly stroke. This acute or post-acute treatment can be performed as a monotherapy or in combination with further drugs, preferably a thrombolytic drug, an anticoagulant or an anti platelet drug, more preferably with t-PA or a variant thereof or DSPA alpha 1.

Since the deleterious effects of the endogenous t-PA also counteract the beneficial effect of an exogenously applied thrombolytic drug, the combined use of the t-PA inhibitor should even improve the outcome of a thrombolytic therapy for thrombotic drugs that are not associated with neurotoxic and/or vasculotoxic side effects, such as e.g. DSPA alpha 1.

Hence the t-PA inhibitor can be given in combination with a thrombolytic drug that does not adversely interact with the BBB or the NMDA receptor or does not possess a deleterious effect on the vasculature or on the neural cells. For such a combination DSPA alpha 1 is preferred.

According to a further embodiment of the invention the thrombolytic drug and the t-PA inhibitor are given in equimolar concentrations.

The composite according to the invention can comprise either a single preparation including a therapeutically effective amount of at least both components (the thrombolytic drug and the t-PA inhibitor) or represents two separate preparations, each containing at least a therapeutically effective amount of one of the components, the thrombolytic drug or the t-PA inhibitor. When two separate preparations are used, they can be administered to the patient either simultaneously or subsequently. Accordingly, both components can either be administered as a combinatorial preparation or concomitantly as separate preparations. Where applicable and suitable, the preparations can be administered as a single bolus or as an infusion, or a combination thereof. Also, multiple consecutive infusions can be applied.

The t-PA inhibitor and the thrombolytic drug, particularly a plasminogen activator, are given preferably non-orally as a parenteral application e.g. by intravenous or subcutaneous application. An intravenous or subcutaneous bolus application is possible. Thus, according to the invention a pharmaceutical composition is provided, which is suitable for parenteral administration.

According to the invention the therapy using a thrombolytic drug, in particular t-PA or variants thereof or DSPA alpha 1 in combination with said t-PA inhibitor comprises one or more of the following characteristics:

(a) the thrombolytic drug, in particular a plasminogen activator, can be administered to the patient more than 3 hours, preferably more than 4.5 hours, preferably more than 6 hours and even more than 9 or 12 hours later than the stroke onset;

(b) the dose of the thrombolytic drug, in particular t-PA, for the treatment can be increased in comparison to the dose that is recommended for the monotherapy with said thrombolytic drug;

(c) the thrombolytic treatment can be applied in patients that are currently not eligible for a thrombolytic therapy, namely patients which suffered the stroke earlier than 3 hours, more preferably earlier than 4,5 hours before presented for the possible treatment or patients with an increased bleeding risk.

Accordingly the invention allows a thrombolytic stroke therapy beyond a 3 hours, preferably beyond a 4.5 hours therapeutic time window, even more preferred beyond 9 or even 12 hours from the onset of stroke.

Thus in one aspect of the invention the t-PA inhibitor is used to reduce, prevent or delay one or more adverse side effects that occur as a result of thrombolytic treatment. These side effects include bleedings such as intracranial or intracerebral bleedings.

In a further embodiment of the invention the application of the t-PA inhibitor allows an increased dose of a thrombolytic drug above the levels given normally in the respective indication, preferably above the doses that are recommended by the manufacturer of the thrombolytic drug.

The invention allows for the use of various thrombolytic drugs. Preferably the thrombolytic drug is a plasminogen activator, more preferably recombinant t-PA (rt-PA, e.g. Alteplase) or variants of t-PA such as Pamiteplase, Lanoteplase, Reteplase, Tenecteplase or Monteplase), urokinase or DSPA variants such as DSPA alpha 1, DSPA alpha 2, DSPA beta or DSPA gamma.

As outlined above the kringle proteins of the invention can be constituted by an inactivated plasminogen activator, preferably selected from the group consisting of DSPA alpha 2, DSPA beta or DSPA gamma, urokinase, Alteplase, or variants of t-PA such as Reteplase, Pamiteplase, Lanoteplase, Reteplase, Tenecteplase or Monteplase.

The variants of DSPA (Desmodus rotundus plasminogen activator) are subject of the US patents US 5, 830, 849 and US 6,008,019, which are fully incorporated herein by reference.

Particularly the inactivated DSPA alpha 1 can be used, wherein it is preferably linked to a suicide substrate, more preferably to D-phenyl-prolyl-arginine chloromethyl ketone (PPACK).

According to this aspect of the invention said kringle protein or peptide includes one or more domains, regions or catalytic sites found in plasminogen activators or related proteins, such as e.g. the finger domain (F) the epidermal growth factor domain (EGF) or the lysine binding site. As a source for this sequence(s) recombinant t-PA (rt-PA, e.g. Alteplase) or variants of t-PA such as Pamiteplase, Lanoteplase, Tenecteplase or Monteplase), urokinase or DSPA variants such as DSPA alpha 1, DSPA alpha 2, DSPA beta or DSPA gamma are preferred. Furthermore domains, regions or catalytic sites from different plasminogen activators proteins can be combined with each other.

The plasminogen activator variants encompass also plasminogen activators with altered or lacking glycosylation, such as e.g. the non-glycosylated recombinant plasminogen activator, Reteplase.

In one aspect of the invention chimeras between tow different plasminogen activators can be used, such as e.g. the chimera between t-PA and the recombinant single chain urokinase (so called rscu-PA). Furthermore fusion proteins between tPA and other proteins, preferably an antibody, might be used. The antibody can target the plasminogen activator or fragment thereof to the thrombus and as a potential target fibrin or the platelets can be choosen. Particularly the antibody is directed against the complement receptor type 1 (CR1).

In one preferred aspect of the invention said kringle protein or peptide consists only of the kringle domain, or a fragment thereof, wherein the fragment comprises at least 8, preferably at least 15 to 30 contiguous amino acids of a kringle domain, wherein said peptide inhibits the t-PA transport across the BBB by at least 20% as assessable according to example A, chapter 2 below.

In a specific aspect of the invention said kringle peptide consists of the kringle domain from, DSPA alpha 2, DSPA beta or DSPA gamma, urokinase, Alteplase, or variants of t-PA such as Reteplase, Pamiteplase, Lanoteplase, Reteplase, Tenecteplase or Monteplase and preferably of the kringle domain from DSPA alpha 1.

Thus the invention further relates to a protein or peptide that comprises

(a) an amino acid sequence according SEQ ID NO:3 (DSPA kringle); or

(b) an amino acid sequence with at least 70% identity, preferably 80%, more preferably 90% and most preferable 95% or 100% identity to the amino acid sequence given in SEQ ID NO:3; or

(c) an amino acid sequence with at least 92% identity, preferable at least 95% or 100% identity to the DARK motif defined by the sequence:

$$\text{``CY-X}_3\text{-G-X}_2\text{–YRGTXS-X}_2\text{-ESR-X}_2\text{-C-X}_2\text{-WNS-X}_2\text{-LXR-X}_2\text{-Y-X}_3\text{-}$$
$$\text{MPXAFN-LGLGXHNYCRNPNXAXKPWCXVXK-X}_3\text{-F-X}_2\text{.ESC-X}_2\text{-PXC''};$$

wherein X denotes an arbitrary amino acid;
and wherein the protein or peptide does not exhibit a serine protease activity and with the proviso that said protein or peptide is not the kringle domain of t-PA or urokinase, and is not the DSPA alpha 1 protein or t-PA covalently linked to a suicide substrate.

According to the invention said t-PA inhibitor can be used as a neuroprotectant.

An additional aspect of the invention provides a pharmaceutical composition selected from the group consisting of one ore more of said t-PA inhitors together with or without a thrombolytic drug, wherein said pharmaceutical composition may further comprise one or more pharmaceutically acceptable carriers or excipients.

In a further aspect of the invention a method of treating a neurological or neurodegenerative disorder, in particular stroke is provided, comprising administering a therapeutically effective amount of said t-PA inhibitor, alone or in combination with therapeutically effective amount of a thrombotic drug, preferably a plasminogen activator, more preferably t-PA.

The term "neurological disorder" as used herein is defined as disease, disorder or condition which directly or indirectly affects the normal functioning or anatomy of a subject's nervous system.

In the context of the invention the term "neurodegenerative disorder" is defined as disease, in which cells of the central or peripheral nervous system are lost.

Examples for neurological and/or neurodegenerative disorders are spinal cord injury, intracranial or intravertebral lesions including, but not limited to, contusion, penetration, shear, compression or laceration lesions of the spinal cord or whiplash shaken infant syndrome.

In the context of the present invention the neurological disorders also include ischaemic events, or ischaemia or ischaemic disorders which can be defined as any local or regional state of hypoxia in cells or tissues which are usually due to an inadequate blood supply (circulation), e.g. caused by a blockage or obstruction of a blood vessel in this area.

The hypoxia can cause acute injury as in hypoxia and/or ischemia including, but not limited to, cerebrovascular insufficiency, cerebral ischemia or cerebral infarction (including cerebral ischemia or infarctions originating from embolic occlusion and thrombosis), retinal ischemia (diabetic or otherwise), glaucoma, retinal degeneration, multiple sclerosis, ischemic optic neuropathy, reperfusion following acute cerebral ischemia, perinatal hypoxic-ischemic injury, or intracranial hemorrhage of any type (including, but not limited to, epidural, subdural, subarachnoid or intracerebral hemorrhage).

Accordingly the term "ischaemic disorder" encompasses thrombotic disorders which include conditions associated with or resulting from thrombosis or a tendency towards thrombosis. These conditions include conditions associated with arterial or venous thrombosis that can be treated with a thrombolytic drug.

The term "t-PA" as used herein includes native t-PA and recombinant t-PA, as well as modified forms of rt-PA that retain the enzymatic or fibrinolytic activities of native t-PA. The enzymatic activity of t-PA can be measured by assessing the ability of the molecule to convert plasminogen to plasmin. The fibrinolytic activity of t-PA may be determined by any *in vitro* clot lysis activity known in the art. Recombinant t-PA has been described extensively in the prior art and is known to the person of skill rt-PA is commercially available as alteplase (Activase® or Actilyse®). Modified forms of rt-PA ("modified rt-PA") have been characterized and are known to those skilled in the art. Modified rt-PAs include, but are not limited to, variants having deleted or substituted amino acids or domains, variants conjugated to or fused with other molecules, and variants having chemical modifications, such as modified glycosylation. Several preferred modified rt-PAs have been described in PCT Publication No. WO93/24635; EP 352,119; EP382174.

In the context of the invention the term "suicide substrate" is defined as a compound that resembles a normal substrate closely enough that it undergoes an enzymatic reaction to a product that inhibits the enzyme. As a substrate analogue it often binds irreversibly to an amino acid of the enzyme, usually the catalytic amino acid, thereby blocking the active site of the enzyme to other molecules and effectively and mostly irreversibly inhibits the enzyme.

The term "stroke" as used herein is used for any event of cerebral ischaemia.

The term "treating" or "treatment" refers to any medical measure for preventing, reducing, mitigating or curing physiological disorders within a mammal, in particular a human, in the need thereof.

A "therapeutically effect amount" is defined as the amount of active ingredient that will reduce the symptoms associated with a neurological or neurodegenerative disease, such as stroke. "Therapeutically effective" also refers to any improvement in disorder severity or the frequency of incidences compared to no treatment. The term "treatment" encompasses either curing or healing as well as mitigation, remission or prevention.

As used herein, the term "neuroprotectant" means an agent that is capable of providing neuroprotection, i.e., protecting a neural entity, such as a neuron, at a site of injury, for example, an ischaemic injury or traumatic injury.

In the context of the invention the term "acute treatment" refers to a short-term medical treatment, usually in a hospital, for patients having an acute illness or injury or recovering from surgery. The term "post-acute treatment" refers to a mid-term medical treatment, usually in a hospital, for patients having an acute illness or injury or recovering from surgery.

**EXAMPLES**

**A. INHIBITION OF T-PA NEUROTOXICTY BY A PROTEIN CONTAINING A PARK DOMAIN.**

MATERIALS AND METHODS

The ability of some proteins comprising the PARK domain (DSPA alpha 1, cDSPA, Reteplase, DSPA alpha 2, DSPA beta, and kringle 2 (K2) of alteplase) to inhibit alteplase trans-BBB transport was demonstrated using the materials and methods outlined below.

DSPA alpha 1, cDSPA, Reteplase, DSPA alpha 2, and DSPA beta were provided by PAION Deutschland GmbH. Reteplase was purchased in a Pharmacy and provided by PAION Deutschland GmbH. kringle 2 (from alteplase) is a compound developed and provided by UMR CNRS 61185/INSERM-Avenir, Caen France.

1. TESTED SUBSTANCES

**DSPAα1**
| | |
|---|---|
| Source: | PAION Deutschland GmbH. |
| Chemical/biological name: | Desmoteplase |
| Molecular weight: | 52 kDa |
| Mode of dissolution: | PBS, distilled water |

**cDSPA (clogged desmoteplase)**
| | |
|---|---|
| Source: | PAION Deutschland GmbH. |
| Chemical/biological name: | inactivated desmoteplase, prepared by exposing DSPA to the suicide substrate, D-phenyl-prolyl-arginine chloromethyl ketone |
| Molecular weight: | 52 kDa |

**DSPAα2**
| | |
|---|---|
| Source: | PAION Deutschland GmbH |
| Molecular weight: | 52 kDa |

**DSPAβ**
| | |
|---|---|
| Source: | PAION Deutschland GmbH |
| Molecular weight: | 48.2 kDa |

**Reteplase**
| | |
|---|---|
| Source: | Rapilysin, pharmacy purchase |
| Chemical/biological name: | Reteplase |
| Molecular weight: | 39 kDa |

**kringle 2 (Recombinant kringle 2 domain of alteplase)**
| | |
|---|---|
| Source: | INSERM-Avenir, Dir D. Vivien |
| Chemical/biological name: | K2alteplase, expressed&purified from *E.coli* |
| Molecular weight: | 9.3 kDa |

**rt-PA (alteplase)**
| | |
|---|---|
| Source: | Boehringer Ingelheim, pharmacy purchase |
| Chemical/biological name: | Recombinant human t-PA |
| Molecular weight: | 70 kDa |

Stock solutions were diluted to obtain the required concentration (0.3 $\mu$M or 10 nM) in Ringer HEPES buffer.

2. DESCRIPTION OF THE *IN VITRO* BLOOD-BRAIN BARRIER MODEL.

2.1 ESTABLISHMENT OF THE MODEL.

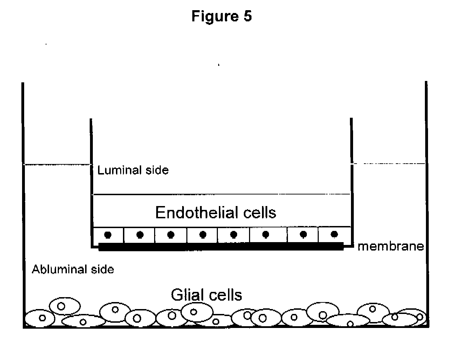

To provide an *in vitro* system for studying brain capillary functions, a co-culture has been developed that closely mimics the *in vivo* situation by culturing brain capillary endothelial cells on one side of a filter and glial cells on the other (figure 5). Endothelial cells were cultured in the upper compartment on a filter while glial cells were maintained in the lower compartment on the plastic of a six-well plate. It is known, that under these conditions, endothelial cells retain all the endothelial markers (factor VIII-related antigen, nonthrombogenic surface, production of prostacyclin, angiotensin converting enzyme activity) and the characteristics of the blood-brain barrier (presence of tight junctions, paucity of pinocytotic vesicles, monoamine oxidase activity, gamma-glutamyltranspeptidase activity and P-glycoprotein).

2.2 CELL CULTURES

Bovine brain endothelial cells were isolated from brain capillaries, grown in DMEM supplemented with 10% (v/v) heat-inactivated calf serum and 10% (v/v) horse serum (Hyclone Laboratories, Logan, UT, USA), 2 mM glutamine,

50 $\mu$g/mL gentamicin and basic fibroblast growth factor (1 ng/mL, added every other day). Sub-clones of endothelial cells frozen at passage 3 were recultured on a 60-mm-diameter gelatin-coated Petri dish and grown to confluency.

Primary glial cultures were isolated from newborn rat cerebral cortex. After removing the meninges, the brain tissue was gently forced through a nylon sieve. DMEM supplemented with 10 % (v/v) fetal calf serum (FCS), 2 mM glutamine, and 50 $\mu$g/mL of gentamycin was used for the dissociation of cerebral tissue and development of glial cells. The glial cells were plated at a concentration of 1.25 x $10^5$ cells ml$^{-1}$ on plastic in six-well plates and incubated at 37°C with 5 % $CO_2$. The medium was changed twice a week. Three weeks after seeding, glial cultures were confluent and composed of astrocytes (~60%), oligodendrocytes and microglial cells.

## 2.3 PREPARATION OF FILTERS

Culture plate inserts (Millicell PC 3 $\mu$m pore size) were coated on the upper side with rat-tail collagen prepared according to the method of Bornstein (1958).

For the analysis of sucrose permeation and compounds tested at 0.3 $\mu$M, filters with a surface of 4.2 cm$^2$ were used. For compounds tested at 10 nM, filters with a surface of 3 cm$^2$ were used.

## 2.4 CO-CULTURE OF BOVINE BRAIN CAPILLARY ENDOTHELIAL CELLS AND GLIAL CELLS.

Coated filters were placed in six-well dishes containing glial cells for co-culture. The co-culture medium was the same as that for brain capillary endothelial cells. Confluent endothelial cells were trypsinized and plated on the upper side of the filters at a concentration of 4·$10^5$ cells/ml. Under these conditions, endothelial cells formed a confluent monolayer within 12 days. Experiments were performed 5 days after confluence.

## 4. TRANSPORT EXPERIMENTS USING THE *IN VITRO* BBB MODEL.

## 4.1 TOXICITY TEST

Sucrose was used as a paracellular marker allowing the evaluation of possible effects of the test compounds on the integrity of the BBB. This small hydrophilic molecule shows a low cerebral penetration and its endothelial permeability coefficient is a measure of the endothelial cell monolayer integrity.

The day of the experiments, Ringer-HEPES was added to the lower compartment (abluminal side) of a six-well plate (2.5 ml per well). One filter containing a confluent monolayer of endothelial cells was transferred to the first well of the plate.

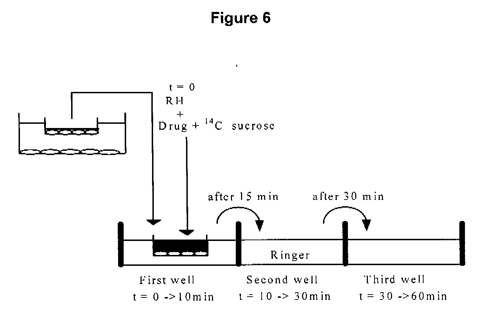

1.5 ml Ringer-HEPES containing the compound in co-incubation with [$^{14}$C]-sucrose (addition of 0.5 $\mu$Ci of [$^{14}$C]-sucrose per filter) was placed in the upper compartment (luminal side). At defined times after addition of the tested compound, the insert was transferred to another well of the six-well plate to minimize the possible reflux of substances from the lower to the upper compartment (refer to figure 6). At the end of each incubation period, aliquots of abluminal and luminal liquid were collected for radioactive analyses.

During the 60-min experiment, the clearance volume increased linearly with time. The average cleared volumes were plotted versus time, and the slope was estimated by linear regression analysis to yield the mean and the associated Standard Error. The slope of the clearance curves for the coculture is denoted $PS_t$, where PS is the permeability x surface area product (in microliters per minute). The slope of the clearance curve for the filter only covered with collagen is denoted $PS_f$.

The PS value for the endothelial monolayer ($PS_e$) was calculated from:

$$\frac{1}{PS_e} = \frac{1}{PS_t} - \frac{1}{PS_f}$$

The $PS_e$ values were divided by the surface area of the filter (4.2 cm$^2$ for Millicell PC and CM and 4.7 cm$^2$ for Transwell inserts) to obtain the endothelial permeability coefficient ($P_e$, in centimeter per minute).

Stability of sucrose permeability was tested with each study compound in triplicate monolayers to confirm the absence of toxic effects on the BBB.

4.2 TRANSPORT OF COMPOUNDS AT 37°C; INTEGRITY TEST

Translocation of rt-PA was determined as described under 4.1 for sucrose.

In these and all other experiments, the integrity of the BBB was monitored using [14C]-sucrose as a paracellular marker, allowing the evaluation of possible effects on the integrity of the BBB.

Data evaluation: The passage of alteplase was evaluated by means of a plasminogen-based zymography assay. It was quantified using an image quantitative system. Moreover, triplicates of luminal and abluminal solutions were assayed for alteplase by means of a spectrozyme assay.

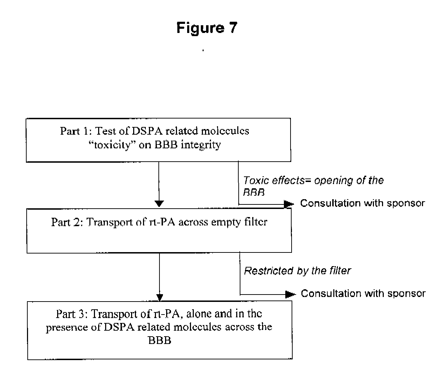

The sequential stages of the study are described in figure 7.

5. TRANSPORT STUDIES

Filter study

In this step, the ability of alteplase to cross the filter was tested to exclude the possibility of transport restriction by the filter. Alteplase was applied at the non-toxic concentration determined in the integrity test.

Transport studies

DSPA-related molecules, reteplase and kringle 2 (alteplase) were studied at equimolar concentrations for their abilities to influence alteplase permeation, evaluated by means of a plasminogen-based zymography assay and quantification by spectrozyme assay.

**EXAMPLE 1: rt-PA in combination with PARK containing proteins exerts no toxic effects on the BBB in vitro.**

Endothelial permeability coefficients obtained with sucrose alone and with alteplase alone or alteplase in combination with a DSPA-related molecule, reteplase or K2 (alteplase) are summarized in the following **table 1.**

**TABLE 1: THE SUCROSE PERMEABILITY WITH OR WITHOUT DSPA-RELATED MOLECULES**

|  | PSt ($\mu$L/min) | Pe (cm.min$^{-1}$) |
| --- | --- | --- |
| Sucrose alone (Control) | 1.21 | $0.32 \times 10^{-3}$ |
| alteplase alone (0.3 $\mu$M) | 1.14 | $0.30 \times 10^{-3}$ |
| alteplase (0.3 $\mu$M) with cDSPA (0.3 $\mu$M) | 1.15 | $0.30 \times 10^{-3}$ |
| alteplase (0.3 $\mu$M) with DSPAalpha1 (0.3 $\mu$M) | 1.06 | $0.28 \times 10^{-3}$ |
| alteplase (0.3 $\mu$M) with DSPAalpha2 (0.3 $\mu$M) | 1.06 | $0.28 \times 10^{-3}$ |
| alteplase (0.3 $\mu$M) with Reteplase (0.3 $\mu$M) | 1.11 | $0.29 \times 10^{-3}$ |
| alteplase (0.3 $\mu$M) with K2 (alteplase) (0.3 $\mu$M) | 0.98 | $0.26 \times 10^{-3}$ |
| alteplase alone (10 nM) | 0.88 | $0.23 \times 10^{-3}$ |
| alteplase (10 nM) with DSPA$\beta$ (10 nM) | 0.87 | $0.22 \times 10^{-3}$ |
| alteplase (10 nM) with DSPA$\gamma$ (10 nM) | 0.81 | $021 \times 10^{-3}$ |

The endothelial permeability coefficients obtained for sucrose showed that alteplase in coincubation with DSPA-related molecules does not exert toxic effects on the *in vitro* BBB at the respective concentrations of 0.3 $\mu$M or 10 nM.

**EXAMPLE 2: Alteplase is able to cross the collagen filter.**

The transport of alteplase (0.3 $\mu$M) through collagen-coated filter was investigated over a period of 120 minutes.

After the experimental period, the concentration of alteplase was determined in luminal and abluminal samples by means of spectrozyme assays (**table 2**).

### TABLE 2: RESULTS FOR THE FILTER STUDY WITH ALTEPLASE (SPECTROZYME ASSAY)

| Filter study | % passage at 120 min |
|---|---|
| alteplase at 0.3 $\mu$M | 7.88 +/- 0.33 § |
| § PERCENTAGE OF ABLUMINAL ALTEPLASE ALONE | |

The results show that alteplase permeation is not restricted by the empty filter, since alteplase showed the potential to cross the filter to an extent of 7.88%. This value was registered as the maximum percentage of diffusional passage through the empty filter and used in the endothelial transport studies as a reference (the so called "versus filter").

**EXAMPLE 3: Transport of rt-PA through the BBB is blocked in vitro by co-incubation with proteins containing a PARK domain.**

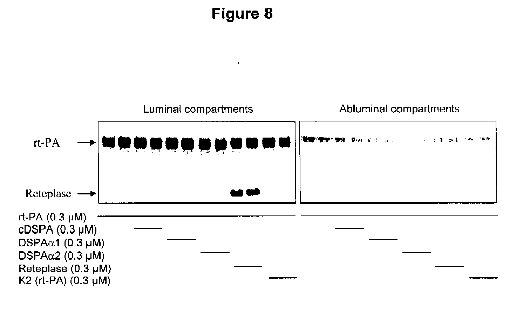

Translocation of alteplase alone and in coincubation with DSPA-related molecules (DSPA$\alpha$1, cDSPA, DSPA$\alpha$2), Reteplase and K2 (alteplase) was studied over a 120 min period. Equimolar concentrations (0.3 $\mu$M) were used.
After the experimental period, the luminal and abluminal concentrations of alteplase were determined by means of a plasminogen containing zymography assay (**figure 8**) and quantified using an image system.
As shown in figure 8, the zymography assay revealed inhibition of alteplase transport across the BBB by cDSPA, DSPA$\alpha$1, DSPA$\alpha$2 and K2 (alteplase) as well as by Reteplase, all of which caused a decrease in the zymography signal. Quantitated data are summarized in **table 3.**

### TABLE 3: INHIBITION OF ALTEPLASE TRANSPORT (ZYMOGRAPHY ASSAY)

| | % of abluminal alteplase |
|---|---|
| Control alteplase at 0.3 $\mu$M | 100 |
| alteplase (0.3 $\mu$M) and cDSPA (0.3 $\mu$M) | 73.3 +/- 1.43 § |
| alteplase (0.3 $\mu$M) and DSPAalpha1 (0.3 $\mu$M) | 46.6 +/- 1.11 § |
| alteplase (0.3 $\mu$M) and DSPAalpha 2 (0.3 $\mu$M) | 25.1 +/- 1.28 § |
| alteplase (0.3 $\mu$M) and Reteplase (0.3 $\mu$M) | 51.6 +/- 1.81 § |
| alteplase (0.3 $\mu$M) and K2 (alteplase) (0.3 $\mu$M) | 75.2 +/- 1.93 § |
| § PERCENTAGE OF ABLUMINAL ALTEPLASE ALONE | |

In a further series, spectrozyme assays were performed in both the luminal and abluminal compartment to quantify the passage of alteplase (0.3 $\mu$M) during coincubation with DSPA-related molecules and K2 (alteplase) (all at 0.3 $\mu$M) (**table 4**).

### TABLE 4: EFFECTS OF POTENTIAL INHIBITORS ON ALTEPLASE TRANSLOCATION ACROSS THE BBB (SPECTROZYME ASSAY); ND : NOT DETERMINED

| | alteplase (% of passage versus filter) §: |
|---|---|
| Control (alteplase at 0.3 $\mu$M) | 100 |
| alteplase (0.3 $\mu$M) and cDSPA | 76.30 +/- 1.30 |
| (0.3 $\mu$M) | |
| alteplase (0.3 $\mu$M) and DSPAalpha1 (0.3 $\mu$M) | 66.95 +/- 1.55 |
| alteplase (0.3 $\mu$M) and DSPAalpha2 (0.3 $\mu$M) | 61.92 +/- 1.75 |
| alteplase (0.3 $\mu$M) and Reteplase (0.3 $\mu$M) | Nd |
| alteplase (0.3 $\mu$M) and K2 (alteplase) (0.3 $\mu$M) | 50.54 +/- 2.15 50.54 +/- 2.15 |
| § PERCENTAGE OF PASSAGE VERSUS FILTER FOR ALTEPLASE ALONE. | |

Because of the protease activity of reteplase the spectrozyme assay could not be used, as it was impossible to distinguish between activities related to alteplase and reteplase.

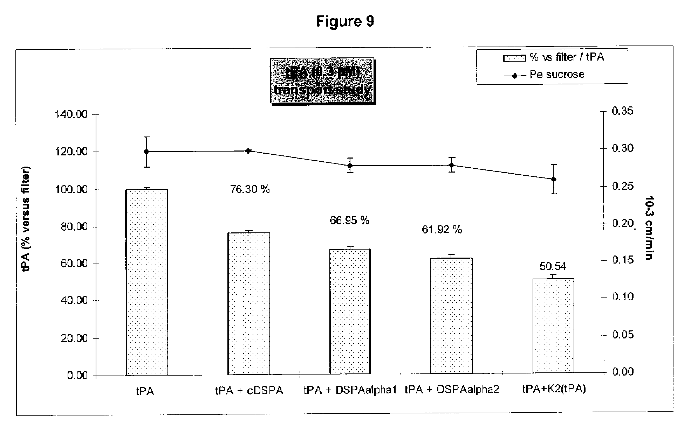

As illustrated in **figure 9**, the spectrozyme assay revealed a decrease in the global proteolytic activity of alteplase in the presence of DSPA-related molecules and K2 (alteplase). Figures are also shown in table 4.

**EXAMPLE 4: DSPA antagonized rt-PA mediated neurotoxic effects after i.v. coadministration but not after coadministration into the striatum.**

The inhibitory effect of a protein comprising a PARK domain was also shown in an animal model as outlined below:

Human recombinant alteplase (rt-PA, alteplase) was from Boehringer Ingelheim (France), NMDA from Tocris (U.K.). Desmoteplase was provided by PAION Deutschland GmbH (Germany).

Animals

Male Sprague Dawley rats (270 to 330 g) were housed in a temperature-controlled room on a 12-hour light/ 12-hour dark cycle, with food and water ad *libitum.* Experiments were performed in accordance with French (act no. 87 to 848; Ministère de l'Agriculture et de la Forêt) and European Communities Council (Directives of November 24, 1986, 86/609/EEC) guidelines for the care and use of laboratory animals.

Striatal Excitotoxic Lesions

Rats were anesthetized with isoflurane (5%, maintenance 2% in oxygen/$N_2O$ (1:3) at 0.8 I/min). Body temperature was maintained at $37 \pm 0.5°C$. Injection pipette (internal diameter 0.32 mm and calibrated at 15 mm/$\mu$l; Hecht Assistent, Germany) was stereotaxically implanted in the right striatum (3.5 lateral and 5.5 ventral to the bregma). NMDA (50 nmol) was injected in a volume of 1 $\mu$l and the pipette was removed 3 minutes later. In the first set of experiments, excitotoxic treatment was complemented after 15 minutes by intravenous injection of alteplase (1 mg/kg), desmoteplase (1 mg/kg), alteplase plus desmoteplase (1 mg/kg each), alteplase vehicle (L-Arg 35 mg/kg, phosphoric acid 10 mg/kg and polysorbate 80, 0.2 %) or desmoteplase vehicle (glycine 4 mg/kg and mannitol 10.64 mg/kg). In the second set of experiments, NMDA was coinjected into the striatum with alteplase (3 $\mu$g), desmoteplase (3 $\mu$g), alteplase plus DSPA (3 $\mu$g each) or the corresponding vehicles, all in a volume of 1 $\mu$l.

Histological Analysis

After 24 hours, rats were euthanized and the whole brain was removed and frozen in isopentane. For volume analysis, one coronal section (20 $\mu$m) out of every 20 was stained with thionine and analyzed, covering the entire lesion. Regions of interest were determined using a stereotaxic atlas for the rat. An image-analysis system (BIOCOM RAG 200, Paris, France) was used to measure the lesion given by the non stained area.

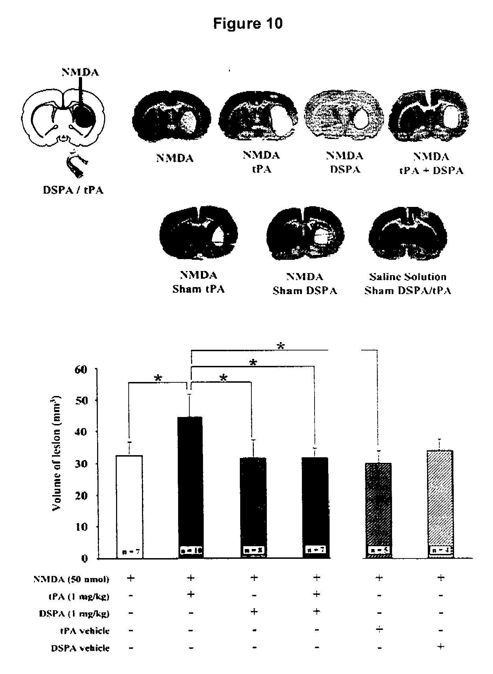

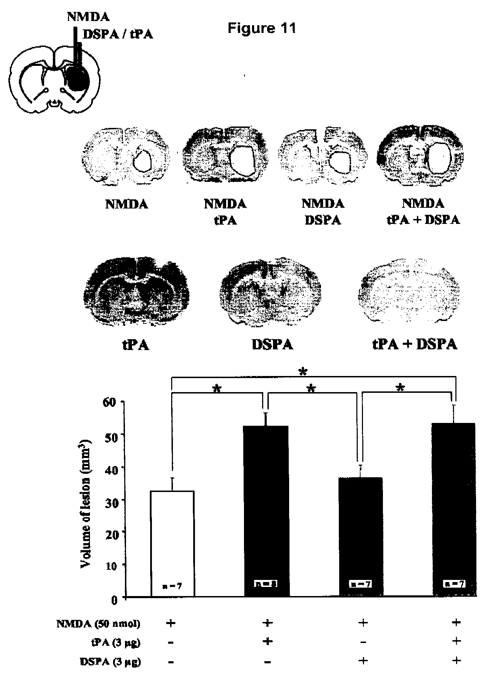

This analysis showed that Desmoteplase antagonized rt-PA mediated neurotoxic effects when both were co-injected intravenously but not when co-administered into the striatum (**figures 10 and 11**), consistent with a relevant *in vivo* competition at the BBB level between these two thrombolytic agents.

**CONCLUSION**

These results show the ability of various proteins comprising a PARK domain to inhibit alteplase trans-BBB transport.

Alteplase alone and in co-incubation with DSPA$\alpha$1, cDSPA, DSPA$\alpha$2, Reteplase and K2 (alteplase), all at a concentration of 0.3 $\mu$M, had no toxic effects on BBB integrity. As analyzed by means of the spectrozyme assay, DSPA$\alpha$1 and DSP$\alpha$2 were able to reduce alteplase transport across the BBB to approximately 67 and 62%, respectively, confirming that DSPA-related molecules can be used to block alteplase blood-to-brain translocation. Similar results (reduction to about 76%) were obtained for clogged DSPA, a DSPA variant that does not exert proteolytic activity. Inhibition was confirmed in zymography quantitation of alteplase.

In addition, isolated kringle 2 was able to reduce alteplase transport by about 50 % (spectroscopy data, confirmed by zymography).

Also Reteplase was able to inhibit alteplase translocation as demonstrated in alteplase zymography analysis. As Reteplase possesses protease activity, its effect could not be quantified by means of the spectrozyme assay.

The molecules tested in this study are able to inhibit rt-PA trans-BBB transport, most likely by competing for LRP-mediated transport. According to the spectrozyme data, they can be ranked as follows: K2 (t-PA) > DSPA$\alpha$2 >

DSPA$\alpha$1 > cDSPA.

In accordance with the results of an in vitro model the kringle domain containing protein was also active ain an in vivo model of neurotoxicity. Herein the plasminogen activator DSAP alpha 1 was able to inhibit the neurotoxic effect of t-PA by preventing its transport across the blood brain barrier.

REFERENCES:

- Benchenane et al., 2005a, Circulation 111 (17): 2241-2249
- Benchenane et al., 2005b, Stroke 36(5): 1065-1070
- Günzler et al. 1982, Hoppe-Seyler's Z. Physiol. Chem. 363(10): 1155-1165
- Pennica, et al. 1983, Nature, 301 (5897): 214-221
- Sottrup-Jensen et al. 1978, in "Progress in Chemical Fibrinolysis and Thrombolysis" (eds. Davidson et al), 3: 191ff
- Walz et al. 1977; Proc. Nat'l. Acad. Sci. USA, 74: 1969-1972
- Yepes et al., 2009; Trends Neurosci 32(1): 48-55

**Legend to figures**

**Figure 1: Computer-based 3D model illustrating conserved and similar amino acids comparable to the PARK domain**. Amino acids highlighted in green represent conserved amino acids, while red displays similar amino acid residues.

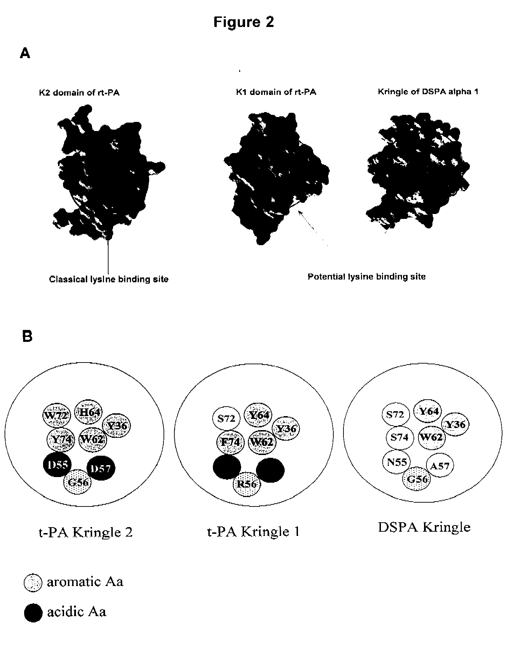

**Figure 2: Binding site of the PARK domain exemplified pictured by kringle of DSPA alpha1, and the kringle 1 and kringle 2 of rt-PA.**

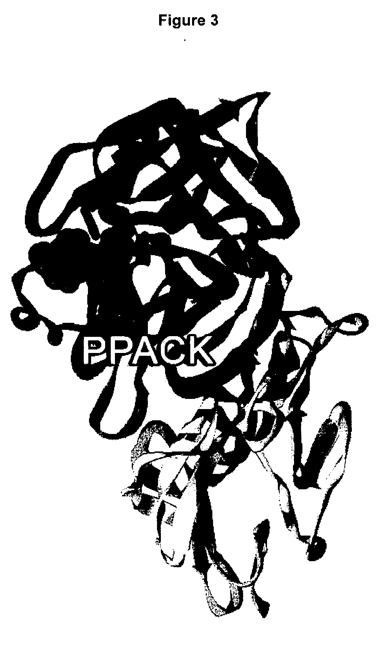

**Figure 3: 3D computer-based model of clogged DSPA alpha 1**. The structure of DSPA after 4400 psec of MD simulation by in silico methods (Swiss Model, GROMOS96, POVRAY) in ribbon representation including the D-phenyl-prolyl-arginine chloromethyl ketone coupled to the catalytic leading to clogged, catalytically inactive DSPA. Light Blue: Finger domain, red: EGF domain; yellow: kringle domain, green: catalytic domain

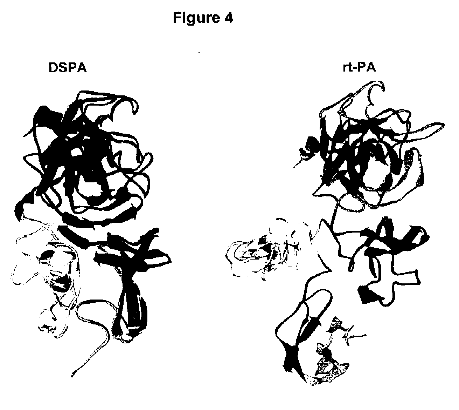

Figure 4: 3D models of alteplase and desmoteplase. The structure of DSPA and rt-PA after 4400 psec and 4000 psec respectively of MD simulation by in silico methods (Swiss Model, GROMOS96, POVRAY) in ribbon representation. Light Blue: Finger domain, red: EGF domain; yellow: kringle domain of DSPA and kringle 1 domain of t-PA; blue: kringle 2 domain of t-PA, green: catalytic domain

**Figure 5: Scheme of the co-culture of endothelial cells and glial cells.**

**Figure 6: Scheme of the permeability studies**

**Figure 7: Experimental procedure of the determination of the BBB transport**

**Figure 8: Zymography assays for rt-PA transport competition (0.3 $\mu$M)** After 2 hours of treatment in the presence of the different compounds indicated, bathing media corresponding to the abluminal compartments (see figure (5) were harvested and subjected to plasminogen and casein containing zymography assays to reveal plasminogen-like activators based on their respective molecular weights and their abilities to activate plasminogen (2 hours of incubation at 37°C) with subsequent casein digestion.

**Figure 9: Bars, lefthand axis: Transport of rt-PA with and without DSPA-related molecules and K2 (t-PA).** The transport was assessed using a spectrozyme assay. Line, righthand axis: unchanged permeability of sucrose (Pe sucrose)

**Figure 10: In vivo antagonism of NMDA-mediated neurotoxicity by DSPA.** Effects of intravenous injection of rt-PA and/or desmoteplase (DSPA) (1 mg/kg each) and their vehicles on the extent of neuronal death induced by the striatal administration of NMDA (50 nmol). *$P$~0.01, ANOVA with Bonferroni correction.

**Figure 11: In vivo antagonism of NMDA-mediated and t-PA enhanced neurotoxicity by DSPA**. Effects of intrastriatal injection of rt-PA and/or desmoteplase (DSPA) (3 µg each) on the extent of neuronal death induced by striatal administration of NMDA (50 nmol). *$P \sim 0.001$, ANOVA with Bonferroni correction

**Claims**

1. Use of a protein or peptide for the manufacture of a medicament for the treatment of neurological or neurodegenerative disorders, in particular stroke, wherein the protein or peptide is selected form the group consisting of a kringle protein or peptide comprising or consisting of

    (i) an amino acid sequence according SEQ ID NO:1, SEQ ID NO:2 or SEQ ID NO:3; or

(ii) an amino acid sequence with at least 70% identity, preferably 80%, more preferably 90% and most preferably 95% identity to the amino acid sequence given in SEQ ID NO:3; or

(iii) an amino acid sequence with at least 90% identity, preferable at least 95% or 100% identity to the plasminogen activator-related kringle (PARK) motif defined by the sequence: "CY-$X_3$-G-$X_2$-YRGTXS-$X_2$-ES-$X_3$-C-$X_2$-WNS-$X_2$-L-$X_4$-Y-$X_4$-PXA-$X_2$-LGLGXHNYCRNP-$X_4$-KPWCXVXK-$X_6$-EXC-$X_2$-PXC", wherein X denotes an arbitrary amino acid; or

(iv) an amino acid sequence with at least 92% identity, preferable at least 95% or 100% identity to the DARK motif defined by the sequence: "CY-$X_3$-G-$X_2$-YRGTXS-$X_2$-ESR-$X_2$-C-$X_2$-WNS-$X_2$-LXR-$X_2$-Y-$X_3$-MPXAFN-LGLGXHNYCRNPNXAXKPWCXVXK-$X_3$-F-$X_2$-ESC-$X_2$-PXC", wherein X denotes an arbitrary amino acid

wherein said protein or peptide does not exhibit a serine protease activity.

2. Use according to claim 1 in combination with a medicament comprising a thrombolytic drug, preferably a plasminogen activator.

3. Use according to claim 2, wherein a therapy using a thrombolytic drug comprises one or more of the following characteristics:

(a) the thrombolytic drug, in particular a plasminogen activator, can be administered to the patient more than 3, preferably more than 4.5 hours, preferably more than 6 hours and even more than 9 or 12 hours later than the stroke onset;

(b) the dose of the thrombolytic drug, in particular t-PA, for the treatment can be increased in comparison to the dose that is recommended for the monotherapy with said thrombolytic drug;

(c) the thrombolytic treatment can be applied in patients that are currently not eligible for a thrombolytic therapy, namely patients which suffered the stroke earlier than 3, more preferably earlier than 4,5 hours before presented for the possible treatment or patients with an increased bleeding risk.

4. Use according to claim 2 or 3, wherein the thrombolytic drug is a plasminogen activator, more preferably recombinant t-PA (rt-PA, e.g. Alteplase) or variants of t-PA such as Pamiteplase, Lanoteplase, Reteplase, Tenecteplase or Monteplase, urokinase or DSPA variants such as DSPA alpha 1, DSPA alpha 2, DSPA beta or DSPA gamma.

5. Use according to one of the above claims, wherein the protein or peptide according to claim 1 is an inactivated plasminogen activator, preferably selected from the group consisting of recombinant t-PA (rt-PA, e.g. Alteplase) or variants of t-PA such as Pamiteplase, Lanoteplase, Reteplase, Tenecteplase or Monteplase), urokinase or DSPA variants such as DSPA alpha 2, DSPA beta or DSPA gamma .

6. Use according to one of the above claims, wherein the protein or peptide according to claim 1 is the inactivated DSPA alpha 1, which are preferably linked to a suicide substrate, more preferably to D-phenyl-prolyl-arginine chloromethyl ketone (PPACK).

7. Use according to one of the above claims, wherein the protein or peptide according to claim 1 includes one or more of further domains found in plasminogen activators, such as the finger domain (F) or the epidermal growth factor domain (EGF), preferably from t-PA or desmoteplase.

8. Use according to one of the above claims, wherein the protein or peptide according to claim 1 consists only of the kringle domain, or a fragment thereof.

9. An isolated kringle protein or peptide comprising or consisting of:

(a) an amino acid sequence according SEQ ID NO:3 (DSPA kringle); or

(b) an amino acid sequence with at least 70% identity, preferably 80%, more preferably 90% and most preferable 95% identity to the amino acid sequence given in SEQ ID NO:3; or

(c) an amino acid sequence with at least 92% identity, preferable at least 95% or 100% identity to the DARK motif defined by the sequence: "CY-$X_3$-G-$X_2$-YRGTXS-$X_2$-ESR-$X_2$-C-$X_2$-WNS-$X_2$-LXR-$X_2$-Y-$X_3$-MPXAFN-LGLGXHNYCRNPNXAXKPWCXVXK-$X_3$-F-$X_2$-ESC-$X_2$-PXC", wherein X denotes an arbitrary amino acid;

wherein the protein or peptide does not exhibit a serine protease activity and with the proviso that said protein or peptide is not the kringle domain of t-PA or urokinase, and not the DSPA alpha 1 protein or t-PA covalently linked to a suicide substrate.

10. Use of a protein or peptide as a neuroprotectant, wherein the protein or peptide is selected form the group consisting of a kringle protein or peptide comprising or consisting of

(i) an amino acid sequence according SEQ ID NO:1, SEQ ID NO:2 or SEQ ID NO:3; or
(ii) an amino acid sequence with at least 70% identity, preferably 80%, more preferably 90% and most preferably 95% identity to the amino acid sequence given in SEQ ID NO:3; or
(v) an amino acid sequence with at least 90% identity, preferable at least 95% or 100% identity to the plasminogen activator-related kringle (PARK) motif defined by the sequence: "CY-$X_3$-G-$X_2$-YRGTXS-$X_2$-ES-$X_3$-C-$X_2$-WNS-$X_2$-L-$X_4$-Y-$X_4$-PXA-$X_2$-LGLGXHNYCRNP-$X_4$-KPWCXVXK-$X_6$-EXC-$X_2$-PXC", wherein X denotes an arbitrary amino acid; or
(vi) an amino acid sequence with at least 92% identity, preferable at least 95% or 100% identity to the DARK motif defined by the sequence: "CY-$X_3$-G-$X_2$-YRGTXS-$X_2$-ESR-$X_2$-C-$X_2$-WNS-$X_2$-LXR-$X_2$-Y-$X_3$-MPXAFN-LGLGXHNYCRNPNXAXKPWCXVXK-$X_3$-F-$X_2$-ESC-$X_2$-PXC", wherein X denotes an arbitrary amino acid

wherein said protein or peptide does not exhibit a serine protease activity.

11. A pharmaceutical composition selected from the group consisting of a protein or peptide according to claim 1 together with or without a thrombolytic drug, wherein said pharmaceutical composition may further comprise one or more pharmaceutically acceptable carriers or excipients.

12. Method of treating a neurological or neurodegenerative disorder, in particular stroke, comprising administering a therapeutically effective amount of an isolated protein or peptide according to claim 10, with or without a therapeutically amount of a thrombotic drug, preferably a plasminogen activator, more preferably t-PA, according to any of the above claims.

## Figure 1

**A**

K2 domain of rt-PA    K1 domain of rt-PA    Kringle of DSPA alpha 1

The lower pictures represent the corresponding kringle domains rotated by 180°

K2 domain of rt-PA    K1 domain of rt-PA    Kringle of DSPA alpha 1

**B**

```
htPA__K1   CYEDQGISYRGTWSTAESGAECTNWNSSALAQRPYSGRRPDAIRLGLGNHNYCRNPDRDS 60
DSPA_K     CYEGQGVTYRGTWSTAESRVECINWNSSLLTRRTYNGRMPDAFNLGLGNHNYCRNPNGAP 60
htPA__K2   CYFGNGSAYRGTHSLTESGASCLPWNSMILIGKVYTAQNPSAQALGLGKHNYCRNPDGDA 60
            ** .;:' ;**** * ;** ..* *** * : '..: '.' ''**;'''''; .

htPA__K1   KPWCYVFKAGKYSSEFCSTPAC 82
DSPA_K     KPWCYVIKAGKFTSESCSVPVC 82
htPA__K2   KPWCHVLKNRRLTWEYCDVPSC 82
            ****;*;*  ; ; * *..* *
```

An asterisk denotes an amino acid that is conserved in all three PARK domains..

# Figure 2

**A**

K2 domain of rt-PA    K1 domain of rt-PA    Kringle of DSPA alpha 1

Classical lysine binding site    Potential lysine binding site

**B**

t-PA Kringle 2    t-PA Kringle 1    DSPA Kringle

aromatic Aa

acidic Aa

**Figure 3**

**Figure 4**

DSPA

rt-PA

## Figure 5

Luminal side

Endothelial cells

membrane

Abluminal side

Glial cells

**Figure 6**

t = 0
R H
+
Drug + $^{14}$C sucrose

after 15 min          after 30 min

Ringer

First well          Second well          Third well
t = 0 ->10min       t = 10 -> 30min      t = 30 ->60min

## Figure 7

```
┌─────────────────────────────────────┐
│  Part 1: Test of DSPA related molecules
│       "toxicity" on BBB integrity    │
└─────────────────────────────────────┘
                    │         Toxic effects= opening of the
                    │         BBB
                    ▼          ───────────► Consultation with sponsor
┌─────────────────────────────────────┐
│  Part 2: Transport of rt-PA across empty filter │
└─────────────────────────────────────┘
                    │         Restricted by the filter
                    │
                    ▼          ───────────► Consultation with sponsor
┌─────────────────────────────────────┐
│  Part 3: Transport of rt-PA, alone and in the
│  presence of DSPA related molecules across the
│                 BBB                  │
└─────────────────────────────────────┘
```

# Figure 8

|  | Luminal compartments | Abluminal compartments |
|---|---|---|
| rt-PA → | | |
| Reteplase → | | |

rt-PA (0.3 µM)
cDSPA (0.3 µM)
DSPAα1 (0.3 µM)
DSPAα2 (0.3 µM)
Reteplase (0.3 µM)
K2 (rt-PA) (0.3 µM)

Figure 9

EP 2 289 541 A1

Figure 10

Figure 11

Europäisches
Patentamt

European
Patent Office

Office européen
des brevets

**PARTIAL EUROPEAN SEARCH REPORT**          Application Number

which under Rule 63 of the European Patent Convention EP 09 01 1145
shall be considered, for the purposes of subsequent
proceedings, as the European search report

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ROUSSEL B D ET AL: "PPACK-Desmodus rotundus salivary plasminogen activator (cDSPA[alpha]1) prevents the passage of tissue-type plasminogen activator (rt-PA) across the blood-brain barrier and neurotoxicity" THROMBOSIS AND HAEMOSTASIS 2009 SCHATTAUER GMBH DEU, vol. 102, no. 3, September 2009 (2009-09), pages 606-608, XP008118874 ISSN: 0340-6245 prepublished online: July 30,2009 * page 606, column 2, line 12 - page 607, column 1, line 14 * * page 608, column 1, line 1 - line 7 * * page 608, column 2, line 5 - line 9 * ----- -/-- | 1-5,7,8, 10-12 | INV. A61K38/49 A61P26/00 C07K14/705 A61P25/00 |

TECHNICAL FIELDS
SEARCHED       (IPC)

A61K
C07K
A61P

### INCOMPLETE SEARCH

The Search Division considers that the present application, or one or more of its claims, does/do
not comply with the EPC to such an extent that a meaningful search into the state of the art cannot
be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Berlin | 24 February 2010 | Schönwasser, D |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 

& : member of the same patent family, corresponding
    document

EPO FORM 1503 03.82 (P04E07)

Europäisches
Patentamt

European
Patent Office

Office européen
des brevets

## PARTIAL EUROPEAN SEARCH REPORT

**Application Number**

EP 09 01 1145

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| X | YI J-S ET AL.: "Infarct reduction in rats following intraventricular administration of either tissue plasminogen activator (tPA) or its non-protease mutant S478A-tPA" EXPERIMENTAL NEUROLOGY, ACADEMIC PRESS, NEW YORK, NY, US, vol. 189, no. 2, 1 October 2004 (2004-10-01), pages 354-360, XP004562956 ISSN: 0014-4886 abstract * page 357, column 2, paragraph 1 - page 358, column 2, line 2; figures 1-3 * * page 359, column 1, paragraph 5 - paragraph 6 * * page 359, column 2, line 18 - line 24 * ----- | 1-5,7, 10-12 | |
| E | WO 2009/116035 A (HADASIT MED RES SERVICE [IL]; HIGAZI ABD [IL]; HIJAZI NUHA [IL]) 24 September 2009 (2009-09-24) * page 6, paragraph 3 - page 8, line 3 * ----- | 1,5,7, 10-12 | TECHNICAL FIELDS SEARCHED (IPC) |
| A | YEPES M ET AL: "Tissue-type plasminogen activator in the ischemic brain: more than a thrombolytic" TRENDS IN NEUROSCIENCE, ELSEVIER, AMSTERDAM, NL, vol. 32, no. 1, 1 January 2009 (2009-01-01), pages 48-55, XP025874693 ISSN: 0166-2236 [retrieved on 2008-10-27] * the whole document * ----- | 1-5,7,8, 10-12 | |

-/--

Europäisches
Patentamt

European
Patent Office

Office européen
des brevets

**PARTIAL EUROPEAN SEARCH REPORT**

Application Number

EP 09 01 1145

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| A | LOPEZ-ATALAYA J P ET AL: "Toward safer thrombolytic agents in stroke: Molecular requirements for NMDA receptor-mediated neurotoxicity"<br>JOURNAL OF CEREBRAL BLOOD FLOW AND METABOLISM 20080630 US,<br>vol. 28, no. 6, 30 June 2008 (2008-06-30), pages 1212-1221, XP002569820<br>ISSN: 0271-678X<br>* the whole document * | 1-5,7,8, 10-12 | |
| | ----- | | |
| A | ROGOVE A D ET AL: "Activation of microglia reveals a non-proteolytic cytokine function for tissue plasminogen activator in the central nervous system"<br>JOURNAL OF CELL SCIENCE 1999 GB,<br>vol. 112, no. 22, 1999, pages 4007-4016, XP002570020<br>ISSN: 0021-9533<br>* page 4008, column 1, paragraph 3 * | 1-5,7,8, 10-12 | TECHNICAL FIELDS SEARCHED (IPC) |
| | ----- | | |

EPO FORM 1503 03.82 (P04C10)

Europäisches
Patentamt

European
Patent Office

Office européen
des brevets

**INCOMPLETE SEARCH
SHEET C**

Application Number

EP 09 01 1145

Although claim 12 is directed to a method of treatment of the
human/animal body (Article 53(c) EPC), the search has been carried out
and based on the alleged effects of the compound/composition.

- - - - -

Europäisches
Patentamt

European
Patent Office

Office européen
des brevets

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-5, 7, 8, 10-12 (all partially)

☐ The present supplementary European search report has been drawn up for those parts of the European patent application which relate to the invention first mentioned in the claims (Rule 164 (1) EPC).

Europäisches
Patentamt
European
Patent Office
Office européen
des brevets

**LACK OF UNITY OF INVENTION
SHEET B**

Application Number

EP 09 01 1145

The Search Division considers that the present European patent application does not comply with the
requirements of unity of invention and relates to several inventions or groups of inventions, namely:

1. claims: 1-5,7,8,10-12 (all partially)

> Use of a protein or peptide for the manufacture of a
> medicament for the treatment of neurological or
> neurodegenerative disorders, wherein the protein or peptide
> is a kringle protein or peptide comprising or consisting of
> an amino acid sequence according to SEQ ID NO:1, wherein
> said protein or peptide does not exhibit a serine protease
> activity; use of a protein or peptide as a neuroprotectant,
> wherein the protein or peptide comprising or consisting of
> the amino acid sequence according to SEQ ID NO:1, wherein
> said protein or peptide does not exhibit a serine protease
> activity; a pharmaceutical composition selected from said
> protein or peptide; method of treating a neurological or
> neurodegenerative disorder, in particular stroke, comprising
> administering a therapeutically effective amount of said
> isolated protein or peptide.
> ---

2. claims: 1-5,7,8,10-12 (all partially)

> Use of a protein or peptide for the manufacture of a
> medicament for the treatment of neurological or
> neurodegenerative disorders, wherein the protein or peptide
> is a kringle protein or peptide comprising or consisting of
> an amino acid sequence according to SEQ ID NO:2, wherein
> said protein or peptide does not exhibit a serine protease
> activity; use of a protein or peptide as a neuroprotectant,
> wherein the protein or peptide comprising or consisting of
> the amino acid sequence according to SEQ ID NO:2, wherein
> said protein or peptide does not exhibit a serine protease
> activity; a pharmaceutical composition selected from said
> protein or peptide; method of treating a neurological or
> neurodegenerative disorder, in particular stroke, comprising
> administering a therapeutically effective amount of said
> isolated protein or peptide.
> ---

3. claims: 1-5,7-9,10-12 (partially), 6 (completely)

Europäisches
Patentamt
European
Patent Office
Office européen
des brevets

**LACK OF UNITY OF INVENTION**
**SHEET B**

Application Number

EP 09 01 1145

The Search Division considers that the present European patent application does not comply with the
requirements of unity of invention and relates to several inventions or groups of inventions, namely:

Use of a protein or peptide for the manufacture of a
medicament for the treatment of neurological or
neurodegenerative disorders, wherein the protein or peptide
is a kringle protein or peptide comprising or consisting of
an amino acid sequence according to SEQ ID NO:3, wherein
said protein or peptide does not exhibit a serine protease
activity; use of a protein or peptide as a neuroprotectant,
wherein the protein or peptide comprising or consisting of
the amino acid sequence according to SEQ ID NO:3, wherein
said protein or peptide does not exhibit a serine protease
activity; a pharmaceutical composition selected from said
protein or peptide; method of treating a neurological or
neurodegenerative disorder, in particular stroke, comprising
administering a therapeutically effective amount of said
isolated protein or peptide.
---

4. claims: 1-5,7,8,10-12 (all partially)

Use of a protein or peptide for the manufacture of a
medicament for the treatment of neurological or
neurodegenerative disorders, wherein the protein or peptide
is a kringle protein or peptide comprising or consisting of
an amino acid sequence according to claim 1(a)(iii), wherein
said protein or peptide does not exhibit a serine protease
activity; use of a protein or peptide as a neuroprotectant,
wherein the protein or peptide comprising or consisting of
the amino acid sequence according to claim 1(a)(iii),
wherein said protein or peptide does not exhibit a serine
protease activity; a pharmaceutical composition selected
from said protein or peptide; method of treating a
neurological or neurodegenerative disorder, in particular
stroke, comprising administering a therapeutically effective
amount of said isolated protein or peptide.
---

5. claims: 1-5,7-9,10-12 (all partially)

| | |
|---|---|
| Europäisches Patentamt<br>European Patent Office<br>Office européen des brevets | **LACK OF UNITY OF INVENTION**<br>**SHEET B** |

Application Number

EP 09 01 1145

The Search Division considers that the present European patent application does not comply with the requirements of unity of invention and relates to several inventions or groups of inventions, namely:

Use of a protein or peptide for the manufacture of a medicament for the treatment of neurological or neurodegenerative disorders, wherein the protein or peptide is a kringle protein or peptide comprising or consisting of an amino acid sequence according to claim 1(a)(iv), wherein said protein or peptide does not exhibit a serine protease activity; use of a protein or peptide as a neuroprotectant, wherein the protein or peptide comprising or consisting of the amino acid sequence according to claim 1(a)(iv), wherein said protein or peptide does not exhibit a serine protease activity; a pharmaceutical composition selected from said protein or peptide; method of treating a neurological or neurodegenerative disorder, in particular stroke, comprising administering a therapeutically effective amount of said isolated protein or peptide.

---

**ANNEX TO THE EUROPEAN SEARCH REPORT
ON EUROPEAN PATENT APPLICATION NO.**

EP 09 01 1145

This annex lists the patent family members relating to the patent documents cited in the above-mentioned European search report.
The members are as contained in the European Patent Office EDP file on
The European Patent Office is in no way liable for these particulars which are merely given for the purpose of information.

24-02-2010

| Patent document cited in search report | Publication date | Patent family member(s) | Publication date |
|---|---|---|---|
| WO 2009116035 A | 24-09-2009 | NONE | |

EPO FORM P0459

For more details about this annex : see Official Journal of the European Patent Office, No. 12/82

## REFERENCES CITED IN THE DESCRIPTION

**Patent documents cited in the description**

- US 5830849 A
- US 6008019 A
- WO 9324635 A
- EP 352119 A
- EP 382174 A

**Non-patent literature cited in the description**

- **Günzler et al.** *Hoppe-Seyler's Z. Physiol. Chem.,* 1982, vol. 363, 1155f
- **Pennica et al.** *Nature,* 1983, vol. 301, 214f
- **Walz et al.** *PNAS,* 1977, vol. 74, 1969f
- **Sottrup-Jensen et al.** Progress in Chemical Fibrinolysis and Thrombolysis. 1978, vol. 3, 191f
- **Benchenane et al.** *Circulation,* 2005, vol. 111 (17), 2241-2249
- **Benchenane et al.** *Stroke,* 2005, vol. 36 (5), 1065-1070
- **Günzler et al.** *Hoppe-Seyler's Z. Physiol. Chem.,* 1982, vol. 363 (10), 1155-1165
- **Pennica et al.** *Nature,* 1983, vol. 301 (5897), 214-221
- **Sottrup-Jensen et al.** Progress in Chemical Fibrinolysis and Thrombolysis. 1978, vol. 3, 191ff
- **Walz et al.** *Proc. Nat'l. Acad. Sci. USA,* 1977, vol. 74, 1969-1972
- **Yepes et al.** *Trends Neurosci,* 2009, vol. 32 (1), 48-55

SEQUENCE LISTING

```
<110>  PAION Deutschland GmbH
       Petersen, Karl-Uwe
       Vivien, Denis

<120>  Treatment of neurological or neurodegenerative disorders

<130>  50 941 K

<160>  3

<170>  PatentIn version 3.3

<210>  1
<211>  82
<212>  PRT
<213>  Homo sapiens


<220>
<221>  Kringle DOMAIN
<222>  (1)..(82)
<223>  t-PA_kringle 1

<400>  1

Cys Tyr Glu Asp Gln Gly Ile Ser Tyr Arg Gly Thr Trp Ser Thr Ala
1               5                   10                  15


Glu Ser Gly Ala Glu Cys Thr Asn Trp Asn Ser Ser Ala Leu Ala Gln
            20                  25                  30


Lys Pro Tyr Ser Gly Arg Arg Pro Asp Ala Ile Arg Leu Gly Leu Gly
        35                  40                  45


Asn His Asn Tyr Cys Arg Asn Pro Asp Arg Asp Ser Lys Pro Trp Cys
    50                  55                  60


Tyr Val Phe Lys Ala Gly Lys Tyr Ser Ser Glu Phe Cys Ser Thr Pro
65                  70                  75                  80


Ala Cys



<210>  2
<211>  82
<212>  PRT
<213>  homo sapiens


<220>
<221>  Kringle DOMAIN
<222>  (1)..(82)
<223>  t-PA_kringle 2

<400>  2

Cys Tyr Phe Gly Asn Gly Ser Ala Tyr Arg Gly Thr His Ser Leu Thr
1               5                   10                  15
```

```
Glu Ser Gly Ala Ser Cys Leu Pro Trp Asn Ser Met Ile Leu Ile Gly
            20              25              30

Lys Val Tyr Thr Ala Gln Asn Pro Ser Ala Gln Ala Leu Gly Leu Gly
        35              40              45

Lys His Asn Tyr Cys Arg Asn Pro Asp Gly Asp Ala Lys Pro Trp Cys
    50              55              60

His Val Leu Lys Asn Arg Arg Leu Thr Trp Glu Tyr Cys Asp Val Pro
65              70              75              80

Ser Cys
```

```
<210>  3
<211>  82
<212>  PRT
<213>  Desmodus rotundus


<220>
<221>  Kringle DOMAIN
<222>  (1)..(82)
<223>  kringle domain of DSPA alpha 1

<400>  3
```

```
Cys Tyr Glu Gly Gln Gly Val Thr Tyr Arg Gly Thr Trp Ser Thr Ala
1               5               10              15

Glu Ser Arg Val Glu Cys Ile Asn Trp Asn Ser Ser Leu Leu Thr Arg
            20              25              30

Arg Thr Tyr Asn Gly Arg Met Pro Asp Ala Phe Asn Leu Gly Leu Gly
        35              40              45

Asn His Asn Tyr Cys Arg Asn Pro Asn Gly Ala Pro Lys Pro Trp Cys
    50              55              60

Tyr Val Ile Lys Ala Gly Lys Phe Thr Ser Glu Ser Cys Ser Val Pro
65              70              75              80

Val Cys
```